# EUROPÄISCHE PATENTSCHRIFT

(11) **EP 0 852 935 B1**
(45) Veröffentlichungstag und Bekanntmachung des Hinweises auf die Patenterteilung: **30.10.2002**
(21) Anmeldenummer: 97120147.0
(22) Anmeldetag: 18.11.1997
(51) Int. Cl.: A61F 5/01, A61F 13/04

(54) **Anatomisch geformte medizinische Bandage**
Anatomically shaped medical bandage
Bandage médical formé anatomiquement

(30) Priorität: 13.12.1996 DE 19651912
(43) Veröffentlichungstag der Anmeldung: 15.07.1998
(73) Patentinhaber: Beiersdorf Aktiengesellschaft, 20245 Hamburg (DE)
(72) Erfinder: Bodenschatz, Stefan, Dr., 21614 Buxtehude (DE); Herzberg, Thorsten, 21149 Hamburg (DE); Doheny, Frank, Co. Tipperary (IE)

(56) Entgegenhaltungen:
- FR-A- 2 615 396
- JP-A- 4 220 255
- US-A- 4 006 542
- US-A- 4 019 505
- US-A- 4 193 395
- US-A- 4 572 167
- DATABASE WPI Week 199238, Derwent Publications Ltd., London, GB; Class A23, AN 1992-313227 'Sheet for medical appliance and rehabilitation' & JP 4 220 255 A (NIRA) 11 August 1992

## Beschreibung

Die Erfindung betrifft medizinische Bandagen, die der Form des Körperteils, an welchem sie angewendet werden sollen, angepaßt sind.

Derartige medizinische Bandagen üben gemäß ihrer Konstruktion und ihrem Indikationsfeld im wesentlichen eine fixierende, führende, stützende oder Druck ausübende Funktion auf das entsprechende Körperteil, wie beispielsweise die Extremitäten, aus. Auch zur Behandlung von Brandwunden werden sie eingesetzt.

Die Herstellung der Bandagen erfolgt durch Ausschneiden von passenden Zuschnitten aus flächigem Material, wie beispielsweise mehr oder weniger elastischen Geweben oder Gewirken, Schaumstoffen, z.B. aus Neopren, und dergleichen. Die anatomiegerechte Form wird dabei durch die Form der Zuschnitte und deren anschließendes Zusammenfügen, gegebenenfalls auch mit zusätzlichen Abnähern und Zwickeln, erreicht, wie es auch sonst bei der Herstellung von Bekleidung üblich ist.

Das Zusammenfügen erfolgt üblicherweise durch Nähen oder Kleben. Der große Nachteil dabei ist, daß eine genaue anatomische Paßform der Bandagen nur schwierig zu erreichen ist und oft eine Vielzahl von Verbindungsstellen, wie Nähten, entstehen. Diese Verbindungsstellen verändern teilweise die Eigenschaften des eingesetzten Materials, z.B. seine elastischen Eigenschaften und die Anschmiegsamkeit, und es besteht vor allem die Gefahr von Druck- oder Scheuerstellen auf der Haut.

Bei Kompressionsstrümpfen und Verbrennungsbandagen tritt dies am häufigsten auf. Die Verbrennungsbandagen weisen zum Beispiel im Bereich der weiblichen Brust oder in der Gesichtspartie eine Vielzahl von Nähten auf, die zu schmerzhaften Druckstellen führen können.

Eine weitere Möglichkeit zur Herstellung von medizinischen Bandagen ist das Formstricken mit Flach- oder Rundstrickmaschinen. Dieses Verfahren ist jedoch von der Möglichkeit der Formgebung und der Materialauswahl her beschränkt, zumal meist nur eine zweidimensionale Formgebung möglich ist und die dritte Dimension ebenfalls durch Verbindungsstellen wie Nähte erzielt werden muß. Zudem ist ein derartiges Verfahren aufwendig.

Es sind auch orthopädische Orthesen bekannt, bei denen Schaumgummi unter Druck zu unterschiedlichen Dicken und Dichten verformt wird. Dadurch können beispielsweise funktionsgerechte Ausformungen vorgenommen und die Eigenschaften des Materials lokal verändert werden (WO 95/32 690). Es handelt sich hierbei jedoch um relativ starre Standard-Produkte und eine genaue Anpassung an die anatomische Form wird nicht erreicht.

Ferner ist es bekannt, thermoplastische Kunststoffplatten formgerecht zu orthopädischen Orthesen und Prothesen zu formen. Das Material, z.B. HDPE oder Polypropylen und dessen Copolymere, besitzen einen thermoplastischen Umformungsbereich von ca. 170 - 250°C und sind nach dem Erkalten weitgehend steif, so daß sie nicht für weiche und anschmiegsame medizinische Bandagen eingesetzt werden können.

JP-A-4220255 offenbart einen Stoff zur medizinischen Anwendung und Rehabilitation, der aus thermoplastischen Polyamidharzen besteht und eine Härte nach Shore D von 25-60 und ein E-Modul von 20-350 N/mm² aufweist. Der Stoff besitzt gute Elastizität und Flexibilität.

Aufgabe der Erfindung war es, die genannten Nachteile zu vermeiden und medizinisch wirksame Bandagen kostengünstig und mit guter Paßform herzustellen.

Gelöst wurde diese Aufgabe durch Bandagen gemäß Anspruch 1.

Als Materialien für die dreidimensionale thermische Verformung eignen sich alle weichen, anschmiegsamen Materialien, aus welchen üblicherweise derartige Bandagen bestehen, sofern sie thermoplastischen Charakter besitzen.

Dies sind insbesondere Vliesstoffe, Gewebe, Gewirke, Gestricke, folienartige Kunststoffe mit geringer Steifigkeit (Rigidität) aus z.B. LDPE, oder Schaumstoffe. Dabei können die Materialien im ganzen aus thermoplastischen Grundstoffen bestehen oder sie können auch nur einen Anteil an derartigen Stoffen enthalten, der aber ausreichend sein muß, um eine gute und stabile Verformbarkeit zu gewährleisten. So können zum Beispiel Vliese, Gewebe, Gewirke und Gestricke nur einen Anteil an thermoplastisch verformbaren Fasern oder Fäden oder anderen Komponenten wie Bindemittel enthalten. Vorteilhaft ist es ferner, wenn diese textilen Materialien in einer oder mehreren Richtungen elastisch ausgebildet sind und deshalb zumindest einen Anteil an dauerelastischen Fasern oder Fäden, wie z.B. Elastan oder Elastodien, enthalten.

Die Materialien können einlagig ausgebildet sein, sie können aber auch aus einem zwei- oder mehrlagigen Laminat bestehen mit thermoplastischem Charakter, beispielsweise aus einem dünnen Schaumstoff oder einer dünnen Kunststoffplatte, die ein- oder beidseitig mit einem Textil kaschiert sind.

Die Materialien sollen weich und anschmiegsam sein, d.h. sie sind in der Regel relativ dünn. Sie können aber auch eine gewisse Dicke von 1 bis mehrere Millimeter aufweisen, so daß sich beispielsweise aus einem thermoplastisch verformbaren Vlies von 5 - 20 mm Dicke hülsen- und schalenartige Teile einer Bandage herstellen lassen.

Die Materialien sollten ferner, um für die vorgesehenen Zwecke geeignet zu sein, einen E-Modul von kleiner als 500 N/mm² (DIN 53 457) und eine Kugeldruckhärte von kleiner als 35 N/mm² (DIN 53 457) aufweisen.

Das thermische Verformen kann auf verschiedene Arten erfolgen. Eine Möglichkeit ist es, die Ausgangsmaterialien bis in den thermoplastischen Erweichungsbereich zu erhitzen und anschließend mit Positivformen, die entsprechend den anatomischen Gegebenheiten geformt sind, zu formen, beispielsweise durch Ansaugen mit Vakuum. Es ist auch möglich, entsprechend gestaltete Positiv- und Negativformen einzusetzen. Eine andere Möglichkeit ist es, das kalte oder vorgewärmte Material in beheizten Formen zu verarbeiten. Durch das Formen kann, z.B. bei Vliesstoffen, das Material zusätzlich ganz oder teilweise komprimiert werden.

Für die Herstellung von Serienprodukten können die Formen üblichen anatomischen Maßen entsprechen. Für Einzelanfertigungen werden die Formen entsprechend den individuellen anatomischen Gegebenheiten gestaltet. Dieser individuellen Formgestaltung kann ein gesondertes Maßschema, ein Formabdruck aus Gips, Wachs und dergleichen oder eine Computer gestützte Maßmethode, z.B. Scannen, zu Grunde gelegt werden.

Die geformten Teile werden anschließend gegebenenfalls mit anderem üblichen Zubehör, wie Klettverschlüssen, Gurten, Schienen usw., zu medizinischen Bandagen zusammengefügt. Die geformten Teile können in umhüllende Materialien, vorzugsweise textile Stoffe, eingearbeitet werden. Es ist aber oft vorteilhaft, daß die Bandagen so ausgebildet sind, daß die geformten Ausgangsmaterialien ohne umhüllendes Material auskommen bzw. verarbeitet werden können.

Durch die Anwendung der Erfindung ist es möglich, medizinische Bandagen herzustellen, die nach den anatomischen Gegebenheiten gestaltet sind. Dies erfolgt durch das thermische Verformen der Ausgangsmaterialien. Auf störende Verbindungsstellen kann weitestgehend verzichtet werden. Die Produkte sind zudem einfach und kostengünstig herzustellen, da sie nicht zur Erreichung der Paßform aus vielen zugeschnittenen Teilen zusammengefügt oder formgestrickt werden müssen.

Die Erfindung wird mit folgenden Beispielen erläutert:

### Beispiel 1

Eine Verbrennungsbandage für das Gesicht besteht aus einem schlauchartigen elastischen Gewirke aus Nylon/Elasthan. Die Bandage ist entsprechend der Anatomie des Kopfes und insbesondere des Gesichtes thermoplastisch geformt. Die zum Formen der Bandage gewählten Formen sind so gestaltet, daß die Bandage nach dem Formen ein definiertes Untermaß hat. Das bedeutet, beim Anlegen muß das elastische Gewirke gedehnt werden, so daß es danach Druck ausübt. Dieser Druck kann sich durch die dreidimensionale Formung gezielt auf der Gesichtspartie verteilen und so wirkungsvoll die Bildung von Keloidsträngen, die häßliche Vernarbungen bilden, vermeiden. Außerdem befinden sich in der Gesichtspartie keine Nähte oder andere Verbindungsstellen, die zu Druckstellen führen könnten.

### Beispiel 2

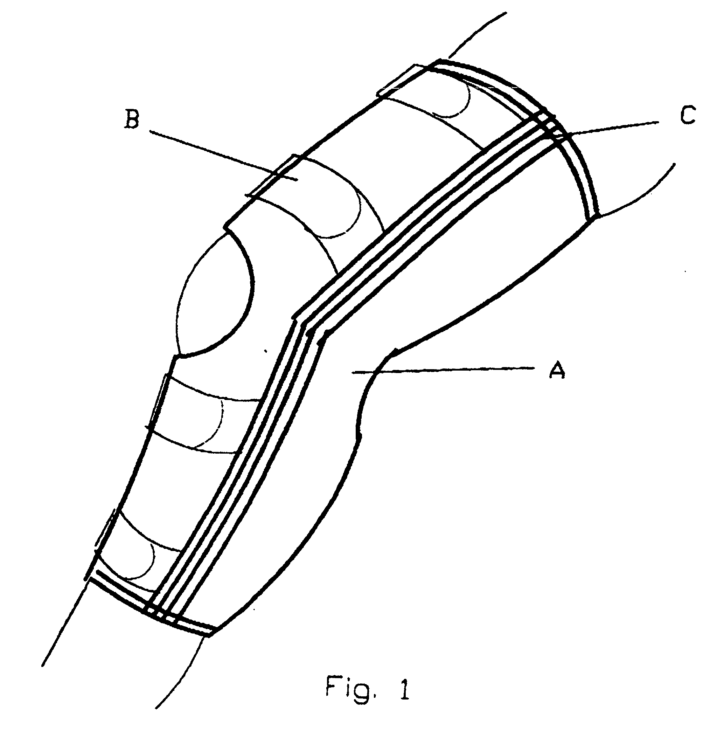

Figur 1 zeigt eine Knieorthese zur Ruhigstellung des Kniegelenks. Die das Bein umschließende Hülse aus einem ca. 10 mm dicken Vliestoff A ist thermisch anatomiegerecht geformt, so daß sie exakt am Bein anliegt. Die Hülse ist mit üblichen Verschlüssen B versehen. Zur besseren Stabilisierung können übliche versteifende Elemente C angebracht sein.

### Beispiel 3

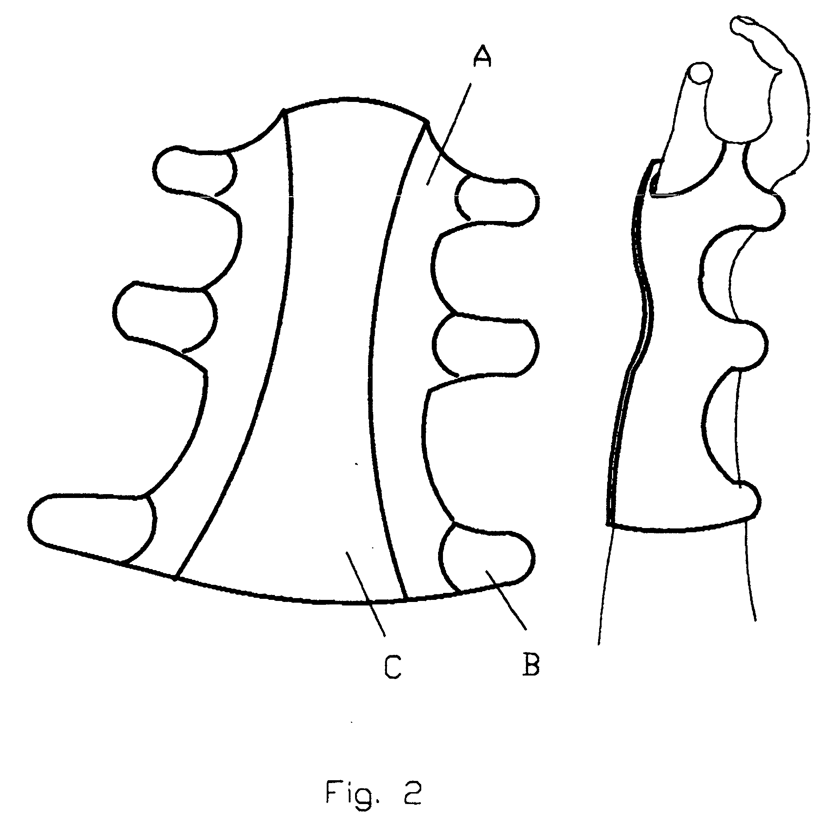

Figur 2 zeigt eine Stützbandage für die Hand, die das Handgelenk bis zum Unterarm umschließt und hülsenartig aus einem ca. 8 mm dicken Vliesstoff A thermisch anatomiegerecht geformt ist. Die Hülse ist mit üblichen Verschlüssen B versehen. Zur besseren Stabilisierung können übliche versteifende Elemente C angebracht sein. Die Bandage ist links in offenem Zustand und rechts in an der Hand angelegtem Zustand dargestellt.

### Beispiel 4

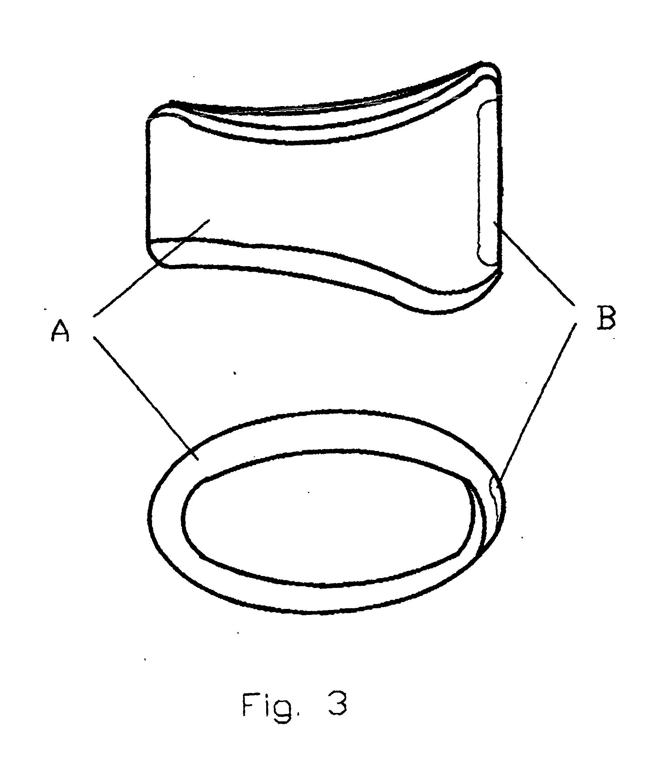

Figur 3 zeigt eine Cervicalstütze aus einem ca. 25 mm dicken anatomisch thermisch geformten Vliesstoff A. Die Cervicalstütze ist mit üblichen Verschlüssen B versehen. Sie ist oben in geschlossenem Zustand von der Seite und unten in geschlossenem Zustand im Querschnitt dargestellt.

## Patentansprüche

1. Medizinische Bandage, die eine dreidimensionale Form aufweist und die der Form des Körperteils, an dem sie zur Anwendung kommt, angepasst ist, **dadurch gekennzeichnet, dass** sie teilweise oder ganz aus einem thermisch verformbaren Material besteht, das im erkalteten Zustand ein E-Modul kleiner 500 N/mm² und eine Kugeldruckhärte kleiner 35 N/mm² aufweist.

2. Medizinische Bandage gemäß Anspruch 1, **dadurch gekennzeichnet, daß** es sich bei dem Material um thermoplastisch verformbaren Vliesstoff, Gewebe, Gewirke, Gestricke, Folie oder Schaumstoff handelt.

3. Medizinische Bandage gemäß Anspruch 2, **dadurch gekennzeichnet, daß** das Material als ganzes thermoplastisch verformbar ist.

4. Medizinische Bandage gemäß Anspruch 2, **dadurch gekennzeichnet, daß** das Material anteilig thermoplastische Fasern oder Komponenten enthält.

5. Medizinische Bandage gemäß mindestens einem der vorhergehenden Ansprüche, **dadurch gekennzeichnet, daß** das Material aus einem zweioder mehrlagigen Laminat besteht, von denen mindestens eine Lage thermisch verformbar ist.

6. Medizinische Bandage gemäß mindestens einem der vorhergehenden Ansprüche, **dadurch gekennzeichnet, daß** die thermisch geformten Teile der Bandage von einem umhüllenden Material umgeben sind.

7. Medizinische Bandage gemäß einem der vorhergehenden Ansprüche erhältlich durch Erwärmung des Materials bis zur thermoplastischen Verformbarkeit und Formung über einer Positivform.

8. Medizinische Bandage nach einem der Ansprüche 1 bis 6 erhältlich durch Erwärmung des Materials bis zur thermoplastischen Verformbarkeit und Formung zwischen einer Positiv- und Negativform.

9. Medizinische Bandage nach einem der Ansprüche 1 bis 6 erhältlich durch Erwärmung bis zur thermoplastischen Verformbarkeit und Formung des Materials in beheizten Formen.

10. Medizinische Bandage gemäß einem der vorhergehenden Ansprüche erhältlich durch thermoplastische Formung des Materials entsprechend den individuellen Körpermaßen.

## Claims

1. Medical bandage which has a three-dimensional shape and is adapted to the shape of the body part on which it is applied, **characterized in that** it consists partially or totally of a thermally deformable material which, in the cooled state, has a modulus of elasticity of less than 500 N/mm² and ball impression hardness of less than 35 N/mm².

2. Medical bandage according to Claim 1, **characterized in that** the material comprises thermoplastically deformable nonwoven fabrics, woven fabrics, knits, film or foam material.

3. Medical bandage according to Claim 2, **characterized in that** the material in its entirety is thermoplastically deformable.

4. Medical bandage according to Claim 2, **characterized in that** the material contains a proportion of thermoplastic fibres or components.

5. Medical bandage according to at least one of the preceding claims, **characterized in that** the material consists of a two-layer or multi-layer laminate, of which at least one layer is thermally deformable.

6. Medical bandage according to at least one of the preceding claims, **characterized in that** the thermally formed parts of the bandage are surrounded by an enclosing material.

7. Medical bandage according to one of the preceding claims, obtainable by heating the material to thermoplastic deformability and forming it using a positive mould.

8. Medical bandage according to one of Claims 1 to 6, obtainable by heating the material to thermoplastic deformability and forming it between a positive mould and negative mould.

9. Medical bandage according to one of Claims 1 to 6, obtainable by heating to thermoplastic deformability and forming the material in heated moulds.

10. Medical bandage according to one of the preceding claims, obtainable by thermoplastically forming the material according to the individual body sizes.

## Revendications

1. Bandage médical, qui présente une forme tridimensionnelle et qui est adapté à la forme de la partie du corps, sur laquelle il est utilisé, **caractérisé en ce qu'**il se compose en tout ou en partie d'un matériau déformable thermiquement, qui présente à l'état refroidi un module E inférieur à 500 N/mm² et une dureté Brinell inférieure à 35 N/mm².

2. Bandage médical suivant la revendication 1, **caractérisé en ce qu'**il s'agit, pour le matériau, d'un non-tissé, d'un tissu, d'un tricot, d'un tissu à mailles, d'une feuille ou d'une mousse, déformable par voie thermoplastique.

3. Bandage médical suivant la revendication 2, **caractérisé en ce que** le matériau est entièrement déformable par voie thermoplastique.

4. Bandage médical suivant la revendication 2, **caractérisé en ce que** le matériau contient des fibres ou des composants partiellement thermoplastiques.

5. Bandage médical suivant au moins une des revendications précédentes, **caractérisé en ce que** le matériau se compose d'un stratifié de deux ou plusieurs couches, dont au moins une couche est déformable thermiquement.

6. Bandage médical suivant au moins une des revendications précédentes, **caractérisé en ce que** les pièces du bandage formées thermiquement sont entourées d'un matériau enveloppant.

7. Bandage médical suivant l'une quelconque des revendications précédentes, réalisable par chauffage du matériau jusqu'à la déformabilité thermoplastique et formage sur une forme positive.

8. Bandage médical suivant l'une quelconque des revendications 1 à 6, réalisable par chauffage du matériau jusqu'à la déformabilité thermoplastique et formage entre une forme positive et une forme négative.

9. Bandage médical suivant l'une quelconque des revendications 1 à 6, réalisable par chauffage jusqu'à la déformabilité thermoplastique et formage du matériau dans des formes chauffées.

10. Bandage médical suivant l'une quelconque des revendications précédentes, réalisable par formage thermoplastique du matériau conformément aux mensurations individuelles du corps.
